# EUROPEAN PATENT APPLICATION

(11) **EP 2 505 657 A1**
(43) Date of publication of application: **03.10.2012**
(21) Application number: 10833295.8
(22) Date of filing: 25.11.2010
(51) Int. Cl.: C12P 19/14

(54) **PROCESS FOR PRODUCTION OF MONOSACCHARIDE**

(30) Priority: 27.11.2009 JP 2009270839
(71) Applicant: Mitsui Chemicals, Inc., Tokyo 105-7117 (JP)
(72) Inventor: MATSUMOTO, Kazuya, Mobara-shi Chiba 297-0017 (JP); OSABE, Masami, Mobara-shi Chiba 297-0017 (JP); FUJII, Ryota, Mobara-shi Chiba 297-0017 (JP); WATANABE, Seiichi, Mobara-shi Chiba 297-0017 (JP); ENDO, Ayako, Mobara-shi Chiba 297-0017 (JP); KIMURA, Sakurako, Mobara-shi Chiba 297-0017 (JP); ARAKI, Tadashi, Mobara-shi Chiba 297-0017 (JP); NAKAYAMA, Akira, Mobara-shi Chiba 297-0017 (JP)
(74) Representative: Wills, Andrew Jonathan
(86) International application number: PCT/JP2010/071066
(87) International publication number: WO 2011/065449

(57) **Abstract**

A monosaccharide production method of producing a monosaccharide from a lignocellulosic raw material comprising: obtaining a saccharified liquid obtained from a lignocellulosic raw material and a saccharification enzyme; recovering the saccharification enzyme from the saccharified liquid by allowing the saccharification enzyme to be adsorbed on the lignocellulosic raw material; and saccharifying the lignocellulosic raw material using the recovered saccharification enzyme.

## Description

### Technical Field

The present invention relates to a method of producing a monosaccharide, especially a method of producing a monosaccharide from a lignocellulosic raw material.

### Background Art

With the recent rise in the awareness of the environment, studies on effective utilization of biomass are actively carried out. Saccharides obtained by degradation of polysaccharides in biomass resources can be utilized for production of various useful substances such as lactic acid, isopropanol, ethanol, and amino acids by using the saccharides as fermentation substrates for microorganisms.

Lignocellulose containing lignin, cellulose obtained by removing lignin from lignocellulose, and the like are generally used as biomass raw materials. Examples of lignocellulosic raw materials include woods such as hardwood and softwood, and agricultural residues such as straw and maize residue. Examples of cellulose from which lignin has been completely removed include cellulose available as a reagent such as Avicel. Further, examples of lignocellulosic raw materials from which lignin has been removed to some extent include hardwood kraft pulp, softwood kraft pulp, mechanical pulp, pulp derived from a herbaceous plant such as kenaf, wastepaper, and a paper sludge containing pulp fibers recovered from paper pulp mills.

Methods for producing monosaccharides, which serve as fermentation substrates, from polysaccharides in biomass resources are roughly classified into two types of method. One of them is an acid saccharification method whereby hydrolysis is effected by a mineral acid, and the other is an enzymatic saccharification method whereby hydrolysis is effected by an enzyme or a microorganism producing the enzyme.
Although the acid saccharification method is technically perfected as compared to the enzymatic saccharification method, the acid saccharification method still incurs a higher cost than a method in which starch, molasses, or the like is used as a raw material. Moreover, an environmental load resulting from disposal of the acid used causes a problem, which prohibits practical use.

In regard to the enzymatic saccharification method, it is known that the high prices of the enzymes, such as cellulase and hemicellulase, prohibit practical use.
It has been tried, as a method for reducing the cost of the enzyme used for saccharification, to chemically or physically pretreat a lignocellulosic raw material as a substrate so as to improve the saccharification reaction efficiency of the enzyme at a later step, thereby reducing the amount of the enzyme to be used. Examples of such a trial include a method whereby a raw material is pretreated with an acid (Japanese Patent Application Laid-Open (JP-A) No. 2008-514207); a pretreatment method whereby a raw material is crushed by a ball mill and thereafter steamed with an acid (JP-A No. 2008-271962); and a pretreatment method whereby cellulose fibers are untangled by beating treatment (JP-A No. 2009-171885). The effect of these substrate pretreatment methods in terms of improving the enzymatic reaction is limited, and is not decisive for cost reduction. For example, in a case in which pulp from which lignin has been highly removed is subjected to beating treatment, the effect in terms of improving the enzymatic reaction is only 1.3-fold (JP-A No. 2009-171885).

Among them, the largest number of reports on application has been made with respect to the pretreatment method using an acid. However, the main object thereof is to remove lignin components from lignocellulosic raw materials, as a result of which a process for separating the acid and lignin is required after the treatment. Although the degree of lignin removal affects the enzymatic reactivity of saccharification enzymes, there has been no report on the enzymatic reactivity in a case in which pretreatment reaction products are not particularly separated after the treatment, or the behavior of the enzymes during the enzymatic reaction thereof.
Moreover, although a method in which the enzyme is recovered after a saccharification reaction and reutilized has been tested in order to further reduce the cost, adherence of the enzyme to solid residues of undegraded substrates hinders the recovery, and stable enzyme recovery has not been successfully carried out.

For example, methods of recovering an enzyme by adsorption and reusing the same, which utilize adsorption of the enzyme onto undegraded substrate solid residues after a saccharification reaction, are known (JP-A No. 59-213396, JP-A No. 62-208282, Japanese Patent No. 1299068, and JP-A No. 63-007781). However, while JP-A No. 59-213396 and JP-A No. 62-208282 describe that the enzyme adsorbed on a substrate solid residue can be utilized as it is, Japanese Patent No. 1299068 and JP-A No. 63-007781 describe that a process of detaching the enzyme from the substrate solid residues is required for recovery and reuse of the enzyme. As discussed above, opinion is divided on the usability of the substrate solid residues after a saccharification reaction. Further, in a case in which the enzyme adsorbed on the substrate solid residues is utilized as it is, the recovered activity varies between 67% and 97%, and only the result of a single recovery is disclosed (JP-A No. 59-213396).

Further, it has been tried to detach the enzyme from the residues by electrization treatment, and recover the enzyme (JP-A No. 2008-206484). However, the operations involved in this method are complicated, and, even if the enzyme recovery is successfully carried out, the method still has a problem in terms of cost. Further, a method of directly recovering an enzyme by treating a saccharification reaction liquid using an ultrafiltration membrane has been tested. Furuichi, et al. proposes a method of recovering an enzyme by treating a saccharification reaction liquid using an ultrafiltration membrane, and then concentrating the saccharide liquid using a reverse osmosis membrane (JP-A No. 58-198299). However, the amount of the enzyme recovered is not disclosed, and a result of a continuous operation is not described, either.

In JP-A No. 2006-87319, it is recognized that adhesion of the enzyme to undegraded residues causes a problem even in a case in which the saccharification reaction liquid is treated using an ultrafiltration membrane, and a continuous saccharification reaction method is disclosed in which the enzyme is separated by filtration using a membrane so as to be retained in the reaction system under a condition in which 96% by mass of the total substrate is saccharified within the retention time. In this case, since an increase of the residues caused by, for example, a decrease in the enzyme activity directly leads to instability of the reaction due to the structure and the operation of the apparatus, the amounts of the enzyme and the substrates are quite limited. In this study of the enzyme recovery employing membrane filtration, it is suggested that, although pulp from which lignin has been highly removed is already used as a substrate, the adsorption of an enzyme on the undegraded residues still poses the largest problem, and reduction thereof is required.

### SUMMARY OF INVENTION

### Problem to be solved by invention

As described above, various techniques for producing saccharides by high-efficiency hydrolysis of fiber components in lignocellulosic raw materials using cellulase or hemicellulase have been developed. However, there still a problem in the high cost of the enzymes required for saccharification, and the economical inefficiency. In order to solve the problem, a method of pretreating a substrate and a method of recovering an enzyme and reusing the same have been tested. However, the former method produces only an insufficient effect, the latter method has a low enzyme recovery ratio, and, therefore, the problem has not yet been solved. The problem of the low enzyme recovery ratio has been considered to be caused by adsorption of the enzyme onto undegraded substrate residues. However, the adsorption to the substrate is difficult to prevent in view of the nature of the enzyme.

Therefore, an object of the present invention is to provide a method of producing a monosaccharide from a lignocellulosic raw material in a simple manner at low cost by allowing reduction of the amount of the enzyme used.

### Means for solving the problem

The present invention comprises the following:
[1] A method of producing a monosaccharide from a lignocellulosic raw material comprising:
   obtaining a saccharified liquid obtained from a lignocellulosic raw material and a saccharification enzyme;
   recovering the saccharification enzyme from the saccharified liquid by allowing the saccharification enzyme to be adsorbed on the lignocellulosic raw material; and
   saccharifying the lignocellulosic raw material using the recovered saccharification enzyme.
[2] The production method as described in [1], wherein the lignocellulosic raw material is hardwood kraft pulp, softwood kraft pulp, mechanical pulp, pulp derived from a herbaceous plant, wastepaper, paper sludge, or any mixture thereof.
[3] The production method as described in [1] or [2], wherein the lignocellulosic raw material is a pretreated raw material obtained by a pretreatment including a heating treatment under an acidic condition followed by neutralization.
[4] The production method as described in [3], wherein a content of hemicellulose in the pretreated raw material is 50% by mass or less of that before the pretreatment.
[5] The production method as described in [3] or [4], wherein furfural in the pretreated raw material is 1% by mass or less of a dry mass of the pretreated raw material.
[6] The production method as described in any one of [1] to [5], wherein the lignocellulosic raw material is a pretreated raw material obtained by pretreatment including the following (a) to (d):
   (a) obtaining a raw material liquid in which a solid of the lignocellulosic raw material has a concentration of from 8% by mass to 30% by mass;
   (b) acidifying the raw material liquid with a mineral acid at a final concentration of from 0.2% by mass to 12% by mass;
   (c) incubating the acidified raw material liquid at from 80°C to 150°C for from 1 hour to 6 hours; and
   (d) adjusting, after the incubating, the raw material liquid so as to have a pH of from 3 to 8 at from 20°C to 80°C.
[7] The production method as described in [6], wherein the mineral acid is sulfuric acid, hydrochloric acid, nitric acid, phosphoric acid, or any combination thereof.
[8] The production method as described in any one of [1] to [7], wherein the recovering comprises the following (e) to (h):
   (e) obtaining a mixture liquid of the saccharified liquid and a lignocellulosic raw material having a solid content mass that is at least 40 times a total protein mass of enzyme active components of the saccharification enzyme;
   (f) incubating the mixture liquid at from 20°C to 80°C for from 1 minute to 3 hours;
   (g) carrying out, after the incubating, solid-liquid separation of the mixture liquid into a saccharified liquid and a lignocellulosic raw material as a solid component on which the enzyme is adsorbed; and
   (h) recovering the separated lignocellulosic raw material as an enzyme-substrate complex.
[9] The production method as described in any one of [1] to [7], wherein the recovering comprises the following (i) to (k):
   (i) terminating a saccharification reaction under a condition in which the lignocellulosic raw material remains in an amount having a solid content mass that is at least 40 times a total protein mass of enzyme active components of the saccharification enzyme;
   (j) carrying out, after the terminating of the reaction, solid-liquid separation of the reaction liquid into a saccharified liquid and an unreacted lignocellulosic raw material as a solid component; and
   (k) recovering the separated lignocellulosic raw material as an enzyme-substrate complex.
[10] The production method as described in [8] or [9], wherein the re-saccharification is carried out by adding a saccharification enzyme of an enzyme active component separated to a liquid side in the solid-liquid separation.
[11] The production method as described in [10], wherein the saccharification enzyme of the enzyme active component separated to the liquid side in the solid-liquid separation includes β-glucosidase.
[12] The production method as described in [8] or [9], wherein a saccharification enzyme of an enzyme active component separated to a liquid side is the enzyme active component separated to the saccharified liquid side in the solid-liquid separation, which is recovered by filtration or purification using a resin.
[13] The production method as described in any one of [1] to [12], wherein the saccharification enzyme is at least one selected from the group consisting of cellulase and hemicellulase.
[14] The production method as described in [13], wherein the cellulase is an enzyme mixture having respective activities of endoglucanase, cellobiohydratase, and β-glucosidase.
[15] The production method as described in [13] or [14], wherein the hemicellulase is an enzyme mixture having respective activities of xylanase, xylosidase, mannanase, pectinase, galactosidase, glucuronidase and arabinofuranosidase.

### BRIEF DESCRIPTION OF DRAWINGS

Fig. 1 is a graph showing results of enzymatic saccharification reactions of a pretreated lignocellulosic raw material according to an example of the present invention, and a lignocellulosic raw material that has not been subjected to the pretreatment.
Figure 2 is a graph showing results of repeated saccharification reactions according to an example of the present invention, in which an enzyme recovered by adsorption on a pretreated lignocellulosic raw material is employed.
Figure 3 is a graph showing results of a reduction in the basic unit for catalyst during repeated saccharification reactions according to an example of the present invention, in which an enzyme recovered by adsorption is employed.
Figure 4 is a graph showing results of repeated saccharification in a case in which an unadsorbed enzyme active component is supplied according to an example of the present invention, and a case which the unadsorbed enzyme active component is not supplied.
Figure 5 is a graph showing results of a reduction in the basic unit for catalyst during repeated saccharification reactions according to an example of the present application, in which an enzyme recovered by using adsorption and membrane is employed.

### BEST EMBODIMENT FOR CARRYING OUT THE INVENTION

A method of producing a monosaccharide according to the present invention is a method of producing a monosaccharide from a lignocellulosic raw material comprising:
obtaining a saccharified liquid obtained from a lignocellulosic raw material and a saccharification enzyme;
recovering the saccharification enzyme from the saccharified liquid by allowing the saccharification enzyme to be adsorbed on the lignocellulosic raw material (hereinafter sometimes referred to as "recovery process"); and
saccharifying the lignocellulosic raw material using the recovered saccharification enzyme (hereinafter sometimes referred to as "re-saccharification process").

The inventors of the present invention have studied a method for reducing the cost by enhancing the recovery ratio of a saccharification enzyme and repeatedly using the saccharification enzyme, and have found that the enzyme component adsorbed on a substrate or an undegraded substrate residue can be reused as it is without carrying out special detachment treatment.
According to the present invention, a saccharification enzyme in the saccharified liquid is recovered by being adsorbed on a lignocellulosic raw material, and the recovered saccharification enzyme is used to saccharify a lignocellulosic raw material so as to obtain a monosaccharide. Therefore, the saccharification enzyme can be recovered in a simple manner, and it is made possible to repeatedly use the saccharification enzyme. As a result of this, it is made possible to greatly reduce the amount of the enzyme used for producing a saccharide, and to produce a monosaccharide from a lignocellulosic raw material in a simple manner at low cost.

In the invention, the term "monosaccharide" primarily means a monosaccharide, which consists of one saccharide unit. A monosaccharide is included in the *monosaccharide* in the invention as long as the monosaccharide is glucose or any other monosaccharide obtained from a lignocellulosic raw material by using a saccharification enzyme. In the invention, however, an oligosaccharide composed of plural sugar units (from 2 to about 100 saccharide units) may be present in a saccharified liquid containing generated monosaccharides.
The scope of the term "process" as used herein includes not only a discrete process, but also a process that cannot be clearly distinguished from another process as long as the expected effect of the process of interest is achieved.
In addition, any numerical range expressed herein using "to" refers to a range including the numerical values before and after "to" as the minimum and maximum values, respectively.
In a case in which the amount of a component that may be included in the composition is indicated in the invention, when there are plural substances corresponding to the component in the composition, the indicated amount means the total amount of the plural substances present in the composition, unless specifically stated otherwise.
The invention will be described below.

In the method of producing a monosaccharide according to the present invention, a saccharified liquid obtained from a lignocellulosic raw material and a saccharification enzyme is used. There is no particular restriction on the saccharified liquid insofar as it is obtained from a lignocellulosic raw material and a saccharification enzyme. The saccharified liquid is preferably obtained by producing a monosaccharide from a lignocellulosic raw material using a saccharification enzyme (saccharification process). In the present invention, an acquired saccharified liquid may be used in the production method, or the saccharification process for obtaining a saccharified liquid may be incorporated as a process of the production method.

The lignocellulosic raw material to be used in the present invention may be any biomass raw material having a low lignin content. The low lignin content refers to a value lower than 30% by mass, which is an average lignin content of biomass raw materials, and is preferably 20% by mass or lower, and more preferably 10% by mass or lower. The raw material having a low lignin content is preferably a fiber that contains cellulose and/or hemicellulose as a major component, and that is obtained by highly removing lignin from a lignocellulosic material such as softwood, hardwood, a logging residue, construction waste wood, a pruning waste, sawdust, kenaf, and an agricultural waste such as rice straw or wheat straw by using a chemical pulp production method such as alkali extraction or alkali digestion or a method such as organosolv. For example, hardwood kraft pulp, softwood kraft pulp, mechanical pulp, pulp derived from a herbaceous plant such as kenaf, wastepaper or paper sludge (including pulp fiber content recovered from a paper pulp mill), or any mixture thereof is more preferable. Such a raw material allows, for example, reliable saccharification treatment with a saccharification enzyme. In particular, hardwood kraft pulp and softwood kraft pulp are preferable for use, and are both available from general pulp manufacturing companies.

The lignocellulosic raw material that serves as a substrate in the enzymatic saccharification method according to the present invention is preferably a pretreated raw material obtained by pretreatment including heating treatment under an acidic condition and subsequent neutralization, and is more preferably a pretreated raw material obtained from the pretreatment of a lignocellulosic raw material having a low lignin content. By the pretreatment of the lignocellulosic raw material, adsorption of the enzyme onto the lignocellulosic raw material can be accelerated.
By the pretreatment including heating under an acidic condition and subsequent neutralization, hemicellulose in a biomass raw material is partly degraded and inverted to a monosaccharide such as xylose. The decrease of the hemicellulose component in the lignocellulosic raw material improves the efficiency of saccharification of the raw material by the enzyme, and also contributes to improvement of the ratio of the subsequent recovery of the enzyme by adsorption.

The pretreated raw material preferably has a hemicellulose content that is 50% by mass or less of the hemicellulose content before the pretreatment. In other words, the degree of the pretreatment is preferably such that 50% by mass or more of the hemicellulose component is degraded to soluble monosaccharides or oligosaccharides. It is more preferable that the amount of furfural as a by-product in the pretreated raw material is limited to be 1% by mass or less of the dry mass of the lignocellulosic raw material. By setting the hemicellulose content to be 50% by mass or less of the hemicellulose content before the pretreatment, the efficiency of the treatment with a saccharification enzyme can be further improved. By suppressing furfural as a by-product to be 1% by mass or less, contamination with microorganisms in the subsequent enzymatic reaction process and the process of recovery of the enzyme by adsorption can be suppressed without significantly affecting the yield in the saccharification reaction liquid. In this regard, furfural, having a relatively low boiling point, can easily be removed by subjecting the generated monosaccharide liquid to vacuum concentration treatment.
When the hemicellulose content is 50% by mass or less of the hemicellulose content before the pretreatment, it is not necessary to completely remove hemicellulose, and 30% by mass or more thereof, or even 40% by mass or more thereof, may remain.

In the pretreatment, first, a lignocellulosic raw material is diluted with water to have a solid content concentration of from 8% by mass to 30% by mass, thereby obtaining a raw material liquid. Since the solubility of a lignocellulosic raw material is quite low, the raw material liquid is prepared in the form of a suspension of solid components. A mineral acid to be used in the pretreatment is selected from sulfuric acid, hydrochloric acid, nitric acid, phosphoric acid, etc., and these may be used singly or in combination. These mineral acids provide, for example, a favorable efficiency of the pretreatment. Among them, sulfuric acid, which is inexpensive, is favorable for industrial use.

The pretreatment includes carrying out acidification by adding a mineral acid to a raw material liquid such that the final concentration of the mineral acid is from 0.2% by mass to 12% by mass, and incubating the acidified raw material liquid at from 80°C to 150°C for from 1 hour to 6 hours. More desirably, it is preferable that the solid of the lignocellulosic raw material has a concentration of from 8% by mass to 20% by mass, a mineral acid is added at a final concentration of 0.2% by mass to 5% by mass, and incubation is carried out at from 90°C to 150°C for from 1 hour to 4 hours. There is no particular restriction on a reaction container to be used for the pretreatment reaction. The pretreatment may be carried out in an acid-resistant heat pressure container, or in an acid-resistant container placed in a heat pressure application apparatus such as an autoclave.

The raw material liquid after the incubation is adjusted to have a pH of from 3 to 8, preferably from 4 to 7, at from 20°C to 80°C, thereby providing a pretreated lignocellulosic raw material (a pretreated raw material). There is no particular restriction on an alkali reagent for the pH adjustment, and NaOH, KOH, ammonia, or the like may be used. The lignocellulosic raw material after the pretreatment still has low solubility, and is recovered in the form of a suspension of solid components.

The saccharification enzyme in the invention means an enzyme which degrades a lignocellulosic raw material to monosaccharide units, and may be any enzyme that degrades a lignocellulosic raw material to monosaccharides, may be any enzyme having activity of cellulase or hemicellulase.
Cellulase may be any enzyme that degrades cellulose to glucose, and examples thereof include those having at least one activity selected from endoglucanase, cellobiohydrolase and β-glucosidase. Cellulase is preferably a mixture of enzymes having the respective activities from the viewpoint of enzyme activity.
Similarly, hemicellulase may be any enzyme that degrades hemicellulose to a monosaccharide such as xylose, and examples thereof include those having at least one activity selected from xylanase, xylosidase, mannanase, pectinase, galactosidase, glucuronidase and arabinofuranosidase. Hemicellulase is preferably a mixture of enzymes having the respective activities from the viewpoint of the enzyme activity.
In the invention, the term "enzyme active component" means each of the saccharification enzymes in the case of an enzyme mixture. In the case of using a single saccharification enzyme, the term "enzyme active component" means the employed saccharification enzyme itself.

There is no restriction on the origins of these cellulases and hemicellulases, and hemicellulase of a filamentous fungus, a basidiomycete, a bacterium or the like may be used. For example, those of various origins such as of the genus *Trichoderma,* the genus *Acremonium,* the genus *Aspergillus,* the genus *Irpex,* the genus *Aeromonas,* the genus *Clostridium,* the genus *Bacillus,* the genus *Pseudomonas,* the genus *Penicillium,* and the genus *Humicola,* and/or those produced by genetic recombination may be used singly, or in mixture of two or more thereof. A commercially-available general cellulase formulation, or a culture of any of the above microorganisms or a filtrate thereof may directly be used as a source of the enzyme. Among them, cellulase having strong cellulose degradation activity, such as cellulase derived from the genus *Trichoderma* or the genus *Acremonium,* is preferable.

Examples of commercially-available cellulases and hemicellulases that can be used include ACCELLERASE 1000 (manufactured by Genencor Inc.), ACCELLERASE 1500 (manufactured by Genencor Inc.), ACCELLERASE XC (manufactured by Genencor Inc.), ACCELLERASE XY (manufactured by Genencor Inc.), CELLUCLAST (manufactured by Novozymes), CELLIC CTEC (manufactured by Novozymes), CELLIC HTEC (manufactured by Novozymes), ACREMONIUM CELLULASE (manufactured by Meiji Seika Kaisha, Limited), MEICELASE (manufactured by Meiji Seika Kaisha, Limited), CELLULASE AMANO A (manufactured by Amano Enzyme Inc.), CELLULASE AMANO T (manufactured by Amano Enzyme Inc.), CELLULASE DAIWA (manufactured by Daiwa Kasei K.K.), CELLULIZER (manufactured by Nagase Biochemicals Ltd.), DRISELASE (manufactured by Kyowa Hakko Kogyo Co. Ltd.), CELLULASE ONOZUKA (manufactured by Yakult Pharmaceutical Industry Co., Ltd.), and CELLULOSIN (manufactured by Hankyu Bioindustry Co., Ltd.). Examples of commercially-available β-glucosidases that can be used include NOVOZYME 188 (manufactured by Novozymes), and ACCELLERASE BG (manufactured by Genencor Inc.).

The saccharification reaction in the present invention is carried out by adding an enzyme to a lignocellulosic raw material and allowing a reaction to proceed while agitating. Although there is no particular restriction on a reaction container to be used for the saccharification reaction, it is preferable that the reaction container is configured such that a lignocellulosic raw material, an enzyme, and the like that are charged therein can be sufficiently mixed by agitation, and has a temperature regulating function that allows the temperature to be maintained at an optimum temperature for the enzyme to be used. The reaction temperature is, for example, preferably from 40°C to 55°C in the case of a commercially-available enzyme originated from a fungus represented by the genus *Trichoderma.* The pH of the liquid in the saccharification reactor is preferably maintained at an optimum pH for the enzyme to be used, and, for example, is preferably from pH4 to pH7 in the case of a commercially-available enzyme originated from the genus *Trichoderma.*
There is no particular restriction on the concentrations of a lignocellulosic raw material as a substrate and an enzyme, which are charged for the reaction. From the viewpoint of handling, such as liquid transfer and charging, of a pretreated lignocellulosic raw material, the solid content concentration is preferably from 8% by mass to 30% by mass. The enzyme to be used may be added in a sufficient amount for efficient degradation of the substrate, in consideration of the activity of the enzyme. The amount of the enzyme may be adjusted appropriately in accordance with, for example, the type of enzyme.

The recovery process in the present invention is preferably carried out as described below. Specifically, the recovery process preferably includes: obtaining a mixture liquid of the saccharified liquid and a lignocellulosic raw material; incubating the mixture liquid for a predetermined time; carrying out, after the incubating, solid-liquid separation of the mixture liquid into a saccharified liquid and a lignocellulosic raw material as a solid component on which the enzyme as adsorbed; and recovering the separated lignocellulosic raw material as an enzyme-substrate complex. This recovery allows 70% by mass or more of the total protein mass of enzyme active components to be recovered by being adsorbed on the solid component.

In this process, the lignocellulosic raw material serves as an enzyme adsorbent rather than as a substrate for the enzyme. In order to ensure that a sufficient amount of the enzyme is adsorbed while avoiding a decrease in the proportion of the saccharide liquid at solid-liquid separation (i.e., maintaining volumetric efficiency), it is preferable to use a lignocellulosic raw material having a solid content mass that is at least 40 times (preferably at least 200 times) the total protein mass of enzyme active components of the saccharification enzyme, and to incubate the mixture liquid for from 1 minute to 3 hours at a temperature at which the stability of the saccharification enzyme can be maintained, for example at from 20°C to 80°C. As a result of the process as described above, a saccharification enzyme in a soluble state can be efficiently adsorbed on a lignocellulosic raw material as a solid component.

As an alternative to a method of newly adding a lignocellulosic raw material, which is to be used for adsorption, into a liquid in which the saccharification reaction has been completed, the saccharification reaction may be terminated before completion, and the saccharification enzyme may be adsorbed on an undegraded substrate residue. Namely, the recovery process may alternatively include: terminating a saccharification reaction under a condition in which the lignocellulosic raw material remains in a predetermined amount; carrying out, after the terminating the reaction, solid-liquid separation of the reaction liquid into a saccharified liquid and an unreacted lignocellulosic raw material as a solid component; and recovering the separated lignocellulosic raw material as an enzyme-substrate complex. As a result of the process as described above, adsorption is carried out directly after the saccharification process, and the enzyme can be recovered in a shorter time.
In the recover process, adsorption of a saccharification enzyme using a pretreated raw material allows the recovery to be carried out efficiently in a shorter time. The incubation time for recovery is preferably from 60 minutes to 120 minutes in the case of using an untreated lignocellulosic raw material that has not been pretreated, and is preferably from 1 minute to 120 minutes in the case of using a pretreated raw material.

Regarding the termination of the reaction, the reaction is preferably terminated at a time when the amount of substrate residues in the reaction container determined by back calculation from the accumulation amount of soluble monosaccharides and oligosaccharides produced by degradation is an amount having a solid content mass that is no more than 200 times the total protein mass of enzyme active components of the saccharification enzyme in the reaction container, and the reaction is more preferably terminated at a time when the amount of substrate residues in the reaction container determined by back calculation from the accumulation amount of soluble monosaccharides and oligosaccharides produced by degradation is an amount having a solid content mass that is at least 40 times the total protein mass of enzyme active components of the saccharification enzyme in the reaction container. Monosaccharide production sufficient as a saccharification process can be achieved by terminating the reaction at a time when the amount of substrate residues is an amount having a solid content mass that is no more than 200 times the total protein mass of enzyme active components of the saccharification enzyme in the reaction container. By setting the amount of substrate residues to have a solid content mass that is at least 40 times the total protein mass of enzyme active components of the saccharification enzyme in the reaction container, it is ensured that the amount of the substrate is sufficient for absorption. Thus, the mass ratio of the substrate residue to the enzyme protein is sufficient, and 70% by mass or more of the enzyme protein can be adsorbed.
Here, the amount of substrate residues in the reaction container may be determined by, for example, measuring the the amount of each of the monosaccharides and oligosaccharides in the mixture liquid or the total quantity thereof using a known analyzer such as high performance liquid chromatography.

The enzyme adsorbed on a lignocellulosic raw material is recovered by subjecting the liquid, which has been subjected to the above-described treatment, to an operation such as centrifuging or coarse-filtrating so as to separate the liquid into a saccharified liquid and a lignocellulosic raw material as a solid component, and collecting the solid component portion. The lignocellulosic raw material on which the enzyme is adsorbed may be employed, as an enzyme-substrate complex, in a saccharification reaction of the next batch.
In regard to the conditions of the centrifugation or the coarse filtration to be applied to the separation of the saccharified liquid and the substrate solid, methods ordinarily used in the relevant industry may be applied as they are. For example, in the case of centrifugation, centrifugation may be carried out at from 500×g to 10,000×g. In the case of coarse filtration, filtration may be carried out with a filter having an aperture of from 0.1 µm to 2 mm and made of stainless steel, ceramic, or resin.

Meanwhile, an enzyme active component distributed to the supernatant saccharified liquid side in the solid-liquid separation process is preferably recovered and used for re-saccharification. By recovering an enzyme component that has not been adsorbed by the above recovery method and adding the recovered enzyme component, a subsequent cycle of the enzymatic saccharification reaction can be performed more stably in the present invention.
The enzyme component that has been distributed to the liquid side is recovered from the supernatant saccharified liquid by filtration with a membrane. Alternatively, the enzyme component that has been distributed to the liquid side may be recovered by purification with a protein purification resin column, and may be employed in a saccharification reaction process of the next batch together with the enzyme component that has been recovered by adsorption on the solid component. Alternatively, the enzyme recovery from the saccharified liquid may not be carried out, in which case the saccharification reaction can be continued with the same activity as in the saccharification reaction process of the previous batch by adding a fresh enzyme component in a corresponding activity amount.

It has been found that the enzyme component that has been distributed to the supernatant saccharified liquid side in the solid-liquid separation process mainly contains β-glucosidase and a part of endoglucanases. Therefore, it is preferable to use β-glucosidase, endoglucanase, or a combination thereof as a saccharification enzyme or saccharification enzymes to be additionally supplied, from the viewpoint of saccharification efficiency.

In the saccharification process after the recovery, a lignocellulosic raw material is saccharified using the saccharification enzyme recovered by the recovery process described above (hereinafter sometimes referred to as "re-saccharification").
Preferably, the enzyme that has been distributed to the liquid side and recovered in the solid-liquid separation process is added. This enables the monosaccharide accumulation ratio to be maintained even when a re-saccharification is carried out repeatedly.

When re-saccharification is carried out using the saccharification enzyme that has been recovered by being adsorbed on a lignocellulosic raw material and the enzyme that has been distributed to the liquid side (which are collectively referred to as "recovered enzyme mixture"), it is preferable to use an enzyme mixture to which a fresh enzyme active component in an activity amount corresponding to the activity amount that has been divided to the saccharified liquid side has been added, as an enzyme component recovered by adsorption on a substrate solid. This allows the enzyme activity amount to be used for re-saccharification to be more accurately controlled and regulated.

Further, it is also preferable to prepare an enzyme mixture by adding an enzyme active component, which has been distributed to the saccharified liquid side in the solid-liquid separation process and recovered by filtration or purification with a resin, to an enzyme component recovered by adsorption on a substrate solid. This removes the necessity to replenish the enzyme, and the amount of the enzyme used can be reduced significantly. The method of collecting the distributed enzyme active component by filtration or purifying the distributed enzyme active component using a resin may employ a membrane apparatus or resin column capable of recovering an enzyme active component, examples of which include a MICROZA pencil-scale module ACP-0013D (manufactured by Asahi Kasei Corporation).

The recovered activity of each enzyme can be determined according to an ordinarily-used method as it is. For example, the activity of β-glucosidase may be a value determined by quantitatively measuring the degradation speed of a cellobiose substrate by an HPLC. The activity of endoglucanase may be a value determined by measuring the degradation activity of an Azo—CM—Cellulose substrate based on a change of absorbance at 590 nm. The enzyme activity recovery ratios can be calculated by comparing these activity values with those of standard enzyme liquids corresponding to the initial concentrations of the enzymes charged.

A lignocellulosic raw material or a pretreated product thereof as a substrate is added to the recovered enzyme mixture, the concentration is adjusted with water so as to match the conditions of the initial saccharification reaction, and a re-saccharification reaction is allowed to proceed under agitation and pH and temperature control.
The sequence of enzymatic saccharification, enzyme recovery, and re-saccharification described above can be carried out repeatedly, and the catalyst cost can be continued to be reduced during a period in which the enzyme activity is maintained.

### EXAMPLES

The present invention will be described in more detail by way of the following examples. However, the examples should not be construed as limiting the present invention. Further, "%" means "% by mass" unless otherwise specified.

### [Example 1] Pretreatment of lignocellulosic raw material

As a lignocellulose-containing material, hardwood kraft pulp (LBKP) was prepared (hereinafter, the terms "lignocellulosic raw material", "pulp" and "LBKP" refers to the same thing and are used interchangeably). LBKP was placed in a glass beaker, and diluted with water to a solid content concentration of 10% [w/w], and then sulfuric acid was added to a final mass concentration of 0.5%. The beaker containing the LBKP-sulfuric acid solution was heated at 130°C in an autoclave for 4 hours. After cooling, the pH was adjusted to 5.0, using NaOH and a citrate buffer solution (at a final concentration of 20 mM), thereby providing a pretreated lignocellulosic raw material. The pretreated lignocellulosic raw material was analyzed by high performance liquid chromatography (HPLC), and it was confirmed that 88% of the hemicellulose component was degraded to soluble xylose, and the amount of furfural produced was as low as 0.5% of the dry mass of the raw material.

### <Analysis conditions>

- Analyzer:: HPLC by JASCO Corporation
- Column:: ULTRON PS-80H (300×8 mm; manufactured by Shinwa Chemical Industries Ltd.)
- Analysis temperature:: 50°C
- Mobile phase:: Perchloric acid aqueous solution at pH 2.1

### [Example 2] Adsorption test of enzyme on lignocellulosic raw material

The adsorption properties of enzymes on the lignocellulosic raw material and the pretreated lignocellulosic raw material were examined respectively. A commercially-available cellulase aqueous solution (Trade name: ACCELLERASE 1000, manufactured by Genencor Inc.), which contains endoglucanase, cellobiohydrolase, β-glucosidase, and hemicellulase, was used as the enzyme. The protein concentration of the cellulase aqueous solution used was measured by the Bradford method, and the measurement result was 2.5% by mass.

Using flasks, the cellulase aqueous solution and the pretreated lignocellulosic raw material were added to a 20 mM citrate buffer solution such that the ratio of (the mass of protein) : (the mass of the solid component of the pretreated lignocellulosic raw material) was 1:13, 1:40, 1:67, 1:120, and 1:200. Each flask was agitated gently at 45°C, and, after 1 minute, the adsorption test liquid was collected and centrifuged at 7,000×g to obtain a supernatant. The protein concentration in the centrifugation supernatant was measured by the Bradford method, and was compared with the protein concentration in the cellulase aqueous solution which had not been subjected to the adsorption treatment.
Similarly, the cellulase aqueous solution and the LBKP (untreated) were added to a 20 mM citrate buffer solution such that the ratio of (the mass of protein) : (the mass of the solid component of the LBKP) was 1:13, 1:40, 1:67, 1:120, and 1:200. The flasks were agitated gently at 45°C, and, after 1 minute, the adsorption test liquid was collected and centrifuged at 7,000×g to obtain a supernatant. The protein concentration in the centrifugation supernatant was measured by the Bradford method, and was compared with the protein concentration in the cellulase aqueous solution which had not been subjected to the adsorption treatment.
The adsorption recovery ratios of the total protein mass of the enzyme active components in the case of using the lignocellulosic raw material and in the case of using the pretreated lignocellulosic raw material according to the above examination are shown in Table 1.

**[Table 1]**

| | | Solid mass ratio of protein mass : Lignocellulosic raw material | | | | |
|---|---|---|---|---|---|---|
| | | 1:13 | 1:40 | 1:67 | 1:120 | 1:200 |
| One minute adsorption | Pretreated | 34% | 74% | 78% | 71% | 91% |
| | Untreated | 0% | 0% | 0% | 0% | 0% |
| 60 minutes adsorption | Pretreated | 19% | 73% | 74% | 75% | 79% |
| | Untreated | 12% | 59% | 67% | 77% | 77% |

### [Example 3] Enzymatic saccharification of lignocellulosic raw material and pretreated product thereof

Untreated pulp having a solid content concentration of 10% by mass and a 20 mM citrate buffer solution (pH 5.0) were added into a separable flask, a cellulase aqueous solution (ACCELLERASE 1000) was added to a final volume concentration of 2% (a final protein concentration of 0.05% by mass), and, thereafter, a saccharification reaction was carried out at 45°C with gentle agitation.

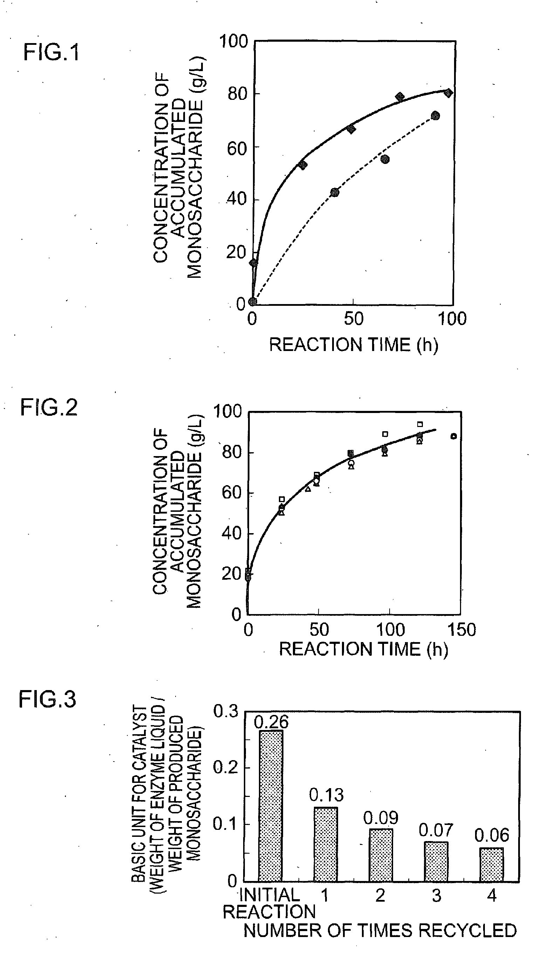

Similarly, the pretreated lignocellulosic raw material (pH 5.0) having a solid content concentration of 10% by mass was added into a separable flask, the cellulase aqueous solution was added to a final volume concentration of 2%, and a saccharification reaction was carried out at 45°C with gentle agitation. The reaction liquid was sampled at certain time intervals, and the amount of monosaccharides produced (the total of glucose and xylose) was analyzed by HPLC in the same manner as in Example 1. The results are shown in Figure 1 and Table 2. The broken line in Figure 1 shows the result of the enzymatic saccharification reaction of the untreated raw material, and the solid line shows the result of the enzymatic saccharification reaction of the pretreated raw material.
As shown in Table 2 and Figure 1, the result of the pretreated lignocellulosic raw material shows a significant increase in the reaction speed, as compared to the result of the enzymatic saccharification reaction of the untreated pulp.

**[Table 2]**

| | | Concentration of Monosaccharide Accumulated(g/L) | |
|---|---|---|---|
| | | Pretreated | Untreated |
| Reaction Time | 0h | 16.3 | 0.2 |
| | 24h | 53.3 | - |
| | 40h | - | 43.0 |
| | 48h | 66.9 | - |
| | 64h | - | 56.0 |
| | 72h | 79.4 | - |
| | 88h | - | 74.2 |
| | 96h | 81.1 | |

### [Example 4] Recovery of enzymes (1)

The enzymes were recovered from the enzymatically saccharified liquid of the pretreated lignocellulosic raw material of Example 3, according to the following method.
To the enzymatically saccharified liquid (reaction liquid after 96 hours of reaction), the pretreated lignocellulosic raw material was added to a final solid content concentration of 2% by mass (which is 40 times the mass of the enzyme protein), and the mixture was agitated gently at 45°C for 1 hour. The resultant adsorption treated liquid was collected and centrifuged at 7,000×g, and the precipitate was collected.
The protein concentration in the centrifugation supernatant was measured by the Bradford method, and it was found that 81% by mass of the enzyme protein was recovered in the precipitate by adsorption. Further, the supernatant was subjected to an SDS-PAGE analysis, and activity analysis based on degradation of substrates such as cellobiose was carried out using HPLC in the same manner as in Example 1. As a result, it was confirmed that the main enzyme component that was not recovered was β-glucosidase, and that 58% of the activity of the β-glucosidase contained in the original cellulase aqueous solution was lost by transfer into the supernatant. The amount of the supernatant monosaccharide liquid was used as monosaccharides produced per 1 reaction in a calculation for the basic unit for catalyst in Figure 3.

### [Example 5] Saccharification (1) of pretreated lignocellulose preparation using recovered enzymes

A re-saccharification reaction was carried out using the precipitate that was recovered in Example 4, namely the pretreated lignocellulosic raw material solid matter on which the enzymes were adsorbed.
The pretreated lignocellulosic raw material solid matter on which the enzymes were adsorbed (having a measured solid content concentration of 20% by mass) and the pretreated lignocellulosic raw material were added into a separable flask, and diluted with a 20 mM citrate buffer solution (pH 5.0) such that the final solid content concentration of the pretreated lignocellulosic raw materials as a total of the both materials became 10% by mass. To this solution, a commercially-available β-glucosidase aqueous solution (Trade name: NOVOZYME 188, manufactured by Novozymes) was added to a final volume concentration of 0.08% (=the activity that was lost by transfer into the supernatant in Example 4), and a re-saccharification reaction was carried out at 45°C with gentle agitation.
The series of enzymatic saccharification, enzyme recovery, and re-saccharification as described in Examples 3, 4, and 5 was repeated 4 times, and the reaction results are shown in Table 3 and Figure 2. In Fig. 2, black diamonds represent the results of the initial reaction, white squares represent the results of the first re-saccharification reaction, black triangles represent the results of the second re-saccharification reaction, white circles represent the results of the third re-saccharification reaction; and white triangles represent the results of the fourth re-saccharification reaction. A decrease of the basic unit for catalyst (=[the mass of the cellulase aqueous solution used + the mass of the β-glucosidase aqueous solution]/[the mass of monosaccharides produced]) in the above case is shown in Figure 3.
As obvious from Figure 2 and Figure 3 as well as Table 3, it is demonstrated that, according to the method of the Examples of the present invention, saccharification can be achieved after enzyme recovery and re-saccharification in nearly the same manner as in the initial reaction, and that a saccharification enzyme can be recycled without impairing the saccharification efficiency.

**[Table 3]**

| | | Concentration of Monosaccharides Accumulated (g/L) | | | | |
|---|---|---|---|---|---|---|
| | | 0 Times | Once | Twice | Three Times | Four Times |
| Reaction Time | 0h | 16.3 | 21.0 | 20.5 | 18.8 | 18.1 |
| | 24h | 53.3 | 56.8 | 52.7 | 52.1 | 50.1 |
| | 42h | - | - | - | - | 62.1 |
| | 48h | 66.9 | 68.7 | 65.8 | 65.7 | - |
| | 72h | 79.4 | 79.6 | 75.0 | 74.2 | 73.5 |
| | 96h | 81.1 | 89.3 | 82.6 | 81.4 | 79.7 |
| | 120h | - | 94.2 | 89.2 | 86.1 | 85.9 |
| | 144h | - | - | - | 88.3 | 88.7 |

### [Example 6] Re-saccharification when β-glucosidase is not added

A saccharification test of the pretreated lignocellulose preparation using the enzymes recovered in Example 5, but without adding the commercially-available β-glucosidase aqueous solution. Namely, the saccharification test was carried out using only the enzymes that had been recovered by adsorption.
The pretreated lignocellulosic raw material solid matter (having a measured solid content concentration of 20% by mass) on which the enzymes were adsorbed, and the pretreated lignocellulosic raw material were added into a flask, and diluted with a 20 mM citrate buffer solution (pH 5.0) such that the final solid content concentration of the pretreated lignocellulosic raw materials (total of the two materials) became 10% by mass. The mixture was then agitated gently at 45°C so as to carry out a re-saccharification reaction, and the amount of produced monosaccharides after 96 hours of reaction was analyzed by HPLC.

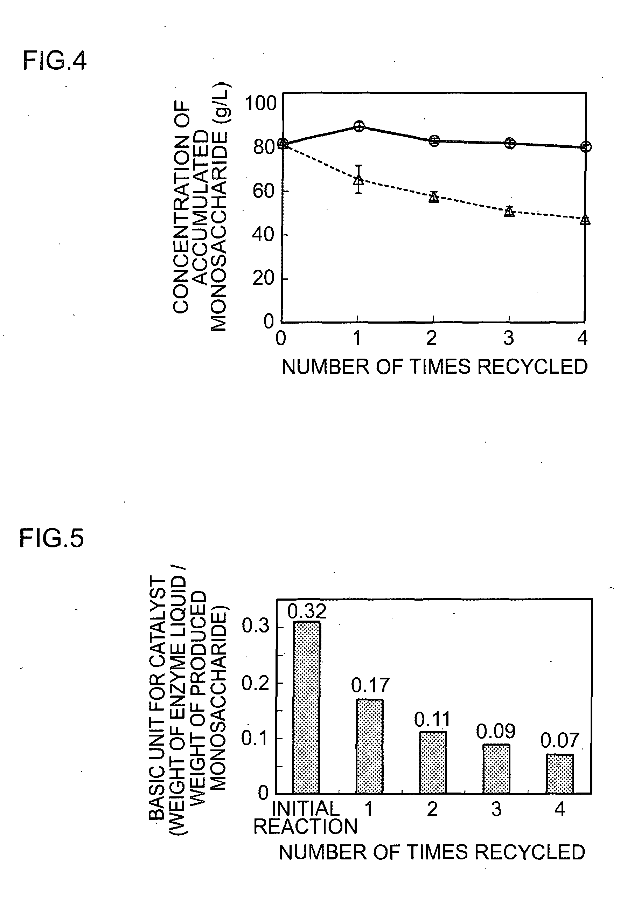

The series of enzymatic saccharification, enzyme recovery, and re-saccharification without addition of β-glucosidase as described in Example 3, Example 4, and Example 6 was repeated 4 times, and changes in the amounts of the produced monosaccharides after 96 hours of incubation in respective cases are shown in Table 4. In Table 4, the broken line represents the results obtained when β-glucosidase was not added, and the solid line represents the results obtained when β-glucosidase was added (Example 5). Each experiment was carried out three times, and an average value thereof is shown together with an error bar thereof. Comparison with the results obtained when the re-saccharification with addition of β-glucosidase according to Example 5 was repeated (the solid line in Figure 4), it is understood that, in a case in which β-glucosidase was not added, the amount of monosaccharides produced decreases as the number of times of the recyclings increases, due to loss of β-glucosidase by transfer into centrifugation supernatants.

### [Example 7] Recovery of enzyme (2)

Enzymatic saccharification of the pretreated lignocellulosic raw material was carried out in the same manner as in Example 3, and the reaction was terminated before the concentration of monosaccharides produced reached 80% by mass based on an analysis using HPLC (the estimated residual substrate solid content concentration being 2% or higher, which is a mass that is 40 times the mass of the enzyme protein). The enzymatically saccharified liquid was centrifuged at 7,000×g, and the precipitate was collected.
The protein concentration in the centrifugation supernatant was measured by the Bradford method, and it was recognized that 74% by mass of the enzyme protein was recovered in the precipitate by adsorption. Further, in order to recover enzymes lost by transfer into the centrifugation supernatant, the centrifugation supernatant liquid was treated with a commercially-available UF membrane (Trade name: MICROZA pencil scale module ACP-0013D, manufactured by Asahi Kasei Corporation), as a result of which 83% by mass of the enzyme protein in the liquid was recovered. It was recognized that 96% by mass of the amount of the initially added enzyme protein was recovered as a total sum of these operations. The amount of the monosaccharide liquid obtained after filtration through the UF membrane was used as the generated monosaccharides per 1 reaction in a calculation of the basic unit for catalyst shown in Figure 5.

### [Example 8] Saccharification (2) of pretreated lignocellulose preparation using recovered enzyme

A re-saccharification reaction was carried out using the enzyme components adsorbed on the pretreated lignocellulosic raw material solid matter recovered in Example 7 as well as the enzyme components recovered from the centrifugation supernatant liquid by filtration through the UF membrane.
The pretreated lignocellulosic raw material solid matter (having a measured solid content concentration of 20% by mass) on which the enzymes were adsorbed, the enzyme liquid obtained by filtration through the UF membrane, and the pretreated lignocellulosic raw material were added into a separable flask, and diluted with a 20 mM citrate buffer solution (pH 5.0) such that the final solid content concentration of the pretreated lignocellulosic raw materials as a total of the materials became 10% by mass. Thereafter, a re-saccharification reaction was allowed to proceed at 45°C under gentle agitation.
The change of the basic unit for catalyst (=[the mass of the cellulase aqueous solution used + the mass of the β-glucosidase aqueous solution]/[the mass of monosaccharides produced]) in a test in which the series of enzymatic saccharification, enzyme recovery, and re-saccharification as described in Examples 7 and 8 was repeated 4 times is shown in Table 4 and Figure 5.

**[Table 4]**

| | | Concentration of Monosaccharides Accumulated | | | | |
|---|---|---|---|---|---|---|
| | | 0 Times | Once | Twice | Three Times | Four Times |
| Added | Concentration | 81.1g/L | 89.3g/L | 82.6g/L | 81.4g/L | 79.7g/L |
| | Standard Deviation | 0.2 | 2.0 | 1.2 | 1.7 | 1.2 |
| Not Added | Concentration | 81.8g/L | 65.2g/L | 57.5g/L | 50.9g/L | 46.9g/L |
| | Standard Deviation | 0.5 | 6.3 | 2.0 | 1.8 | 0.9 |

As shown in Table 4 and Figure 5, it has been found that, by adding an enzyme, a decrease in the saccharification efficiency is prevented when saccharification is repeated, and saccharification enzymes can be utilized effectively from both of the viewpoints of cost and operation efficiency.

Therefore, according to the present invention, the amount of enzyme used can be reduced, and a monosaccharide can be produced from a lignocellulosic raw material in a simple manner at low cost.

The disclosure of Japanese Patent Application No. 2009-270839, filed November 27, 2009, is incorporated herein by reference in its entirety.
All documents, patent applications, and technical standards described in the present specification are herein incorporated by reference to the same extent as if each individual document, patent application, or technical standard was specifically and individually indicated to be incorporated by reference.

## Claims

1. A method of producing a monosaccharide from a lignocellulosic raw material comprising:
obtaining a saccharified liquid obtained from a lignocellulosic raw material and a saccharification enzyme;
recovering the saccharification enzyme from the saccharified liquid by allowing the saccharification enzyme to be adsorbed on the lignocellulosic raw material; and
saccharifying the lignocellulosic raw material using the recovered saccharification enzyme.

2. The production method according to claim 1, wherein the lignocellulosic raw material is hardwood kraft pulp, softwood kraft pulp, mechanical pulp, pulp derived from a herbaceous plant, wastepaper, paper sludge, or any mixture thereof.

3. The production method according to claim 1 or claim 2, wherein the lignocellulosic raw material is a pretreated raw material obtained by pretreatment including heating treatment under an acidic condition followed by neutralization.

4. The production method according to claim 3, wherein a content of hemicellulose in the pretreated raw material is 50% by mass or less of that before the pretreatment.

5. The production method according to claim 3 or claim 4, wherein furfural in the pretreated raw material is 1% by mass or less of a dry mass of the pretreated raw material.

6. The production method according to any one of claim 1 to claim 5, wherein the lignocellulosic raw material is a pretreated raw material obtained by pretreatment including the following (a) to (d):
(a) obtaining a raw material liquid in which a solid of the lignocellulosic raw material has a concentration of from 8% by mass to 30% by mass;
(b) acidifying the raw material liquid with a mineral acid at a final concentration of from 0.2% by mass to 12% by mass;
(c) incubating the acidified raw material liquid at from 80°C to 150°C for from 1 hour to 6 hours; and
(d) adjusting, after the incubating, the raw material liquid so as to have a pH of from 3 to 8 at from 20°C to 80°C.

7. The production method according to claim 6, wherein the mineral acid is sulfuric acid, hydrochloric acid, nitric acid, phosphoric acid, or any combination thereof.

8. The production method according to any one of claim 1 to claim 7, wherein the recovering comprises the following (e) to (h):
(e) obtaining a mixture liquid of the saccharified liquid and a lignocellulosic raw material having a solid content mass that is at least 40 times a total protein mass of enzyme active components of the saccharification enzyme;
(f) incubating the mixture liquid at from 20°C to 80°C for from 1 minute to 3 hours;
(g) carrying out, after the incubating, solid-liquid separation of the mixture liquid into a saccharified liquid and a lignocellulosic raw material as a solid component on which the enzyme is adsorbed; and
(h) recovering the separated lignocellulosic raw material as an enzyme-substrate complex.

9. The production method according to any one of claim 1 to claim 7, wherein the recovering comprises the following (i) to (k):
(i) terminating a saccharification reaction under a condition in which the lignocellulosic raw material remains in an amount having a solid content mass that is at least 40 times a total protein mass of enzyme active components of the saccharification enzyme;
(j) carrying out, after the terminating of the reaction, solid-liquid separation of the reaction liquid into a saccharified liquid and an unreacted lignocellulosic raw material as a solid component; and
(k) recovering the separated lignocellulosic raw material as an enzyme-substrate complex.

10. The production method according to claim 8 or claim 9, wherein the saccharification is carried out by adding a saccharification enzyme of an enzyme active component separated to a liquid side in the solid-liquid separation.

11. The production method according to claim 10, wherein the saccharification enzyme of the enzyme active component separated to the liquid side in the solid-liquid separation includes β-glucosidase.

12. The production method according to claim 8 or claim 9, wherein a saccharification enzyme of an enzyme active component separated to a liquid side is the enzyme active component separated to the saccharified liquid side in the solid-liquid separation, which is recovered by filtration or purification using a resin.

13. The production method according to any one of claim 1 to claim 12, wherein the saccharification enzyme is at least one selected from the group consisting of cellulase and hemicellulase.

14. The production method according to claim 13, wherein the cellulase is an enzyme mixture having respective activities of endoglucanase, cellobiohydrolase, and β-glucosidase.

15. The production method according to claim 13 or claim 14, wherein the hemicellulase is an enzyme mixture having respective activities of xylanase, xylosidase, mannanase, pectinase, galactosidase, glucuronidase and arabinofuranosidase.
